# EUROPEAN PATENT SPECIFICATION

(11) **EP 2 408 317 B1**
(45) Date of publication and mention of the grant of the patent: **04.10.2017**
(21) Application number: 10710401.0
(22) Date of filing: 12.02.2010
(51) Int. Cl.: A23K 20/24, A23K 50/20

(54) **DIETARY SUPPLEMENT**
NAHRUNGSERGÄNZUNGSMITTEL
COMPLÉMENT ALIMENTAIRE

(30) Priority: 17.03.2009 GB 0904584
(43) Date of publication of application: 25.01.2012
(73) Proprietor: Greenteam Holdings Limited, Nailsworth Stroud Gloucestershire GL6 0BS (GB)
(72) Inventor: GREEN, Malcolm, Geoffrey, Gloucestershire GL6 0BS (GB)
(74) Representative: Donald, Jenny Susan
(86) International application number: PCT/GB2010/050231
(87) International publication number: WO 2010/106351

(56) References cited:
- FR-A1- 2 625 415
- US-A1- 2008 014 304
- Anonymous: "Finish Line Thia-cal" 30 December 2005 (2005-12-30), XP002579592 Retrieved from the Internet: URL:http://www.doversaddlery.com/images/pd f/22068info.jpg [retrieved on 2010-04-26]
- Anonymous: "Humavyte" Internet Citation 19 July 2008 (2008-07-19), XP002579590 Retrieved from the Internet: URL:http://web.archive.org/web/20080719131 829/http://www.ranvet.com.au/liquid_chelat ed_minerals.htm [retrieved on 2010-04-23]
- HUDSON N P H ET AL: "Primary hypoparathyroidism in two horses" AUSTRALIAN VETERINARY JOURNAL, vol. 77, no. 8, August 1999 (1999-08), pages 504-508, XP002579591 ISSN: 0005-0423
- GRUBB TAMARA L ET AL: "Hemodynamic effects of calcium gluconate administered to conscious horses" JOURNAL OF VETERINARY INTERNAL MEDICINE, vol. 10, no. 6, 1996, pages 401-404, XP002579708 ISSN: 0891-6640

## Description

### DESCRIPTION OF THE INVENTION

The present invention relates to a dietary supplement. In particular, the present invention relates to a horse supplement for calming horses.

There is a long existing problem that many horses display agitated behaviour and appear to be in a state of anxiety. Such horses often exhibit behavioural problems such as inability to concentrate, difficulty learning and poor sporting performance. It may be that a horse generally demonstrates such agitated behaviour or only does so in certain situations, such as in large crowds or on hearing a loud noise. This can be a particular problem when a horse is taking part in an equestrian sporting event.

Other horses may exhibit this behaviour in recreational situations such as hacking or schooling when safety and rider confidence become significant issues.

There is a general need to be able to calm horses when they are in such an agitated state. Moreover, there is a further need to ensure that the horse maintains a calm temperament yet still has the ability to concentrate, learn and make split second judgements and decisions. This is of particular importance for horses that take part in equestrian sporting events.

Many attempts have been made to improve the temperament of such horses. These attempts include endeavouring to train the horses to maintain a calm temperament in all situations. Although some horses have shown an improved temperament with such training, there remain some horses in which little or no improvement is seen.

Instead of, or as well as, endeavouring to train a horse, another attempt to improve the temperament of a horse is to alter the nutritional intake of the horse. There are a number of dietary supplements on the market that attempt to deal with these problems that are aimed at calming horses. For example some supplements include magnesium as a calming agent. Such supplements have been shown to improve the temperament of some horses yet provide no or little effect on other horses. It is believed that those horses that have been successfully treated had a magnesium deficiency. Other supplements include tryptophan, B group vitamins and herbs, which have shown little or no improvement in the temperament of some horses.

Therefore, it is clear that the temperament of some horses cannot be calmed with the above attempts and there remains a need to improve the temperament of horses in an agitated state.

Calcium coordinated compounds such as calcium gluconate and calcium borogluconate are further known to be intravenously administrated to horses for the treatment of hypocalcaemia, resulting in a decrease in heart rate, see for example Grubb et al., Journal of Veterinary Internal Medicine, 1996, Vol. 10, No. 6, 401-404 or Hudson et al., Australian Veterinary Journal, 1999, Vol. 77, No. 8, 504-508. As will be appreciated there is a need to provide new products, compositions or agents which can calm horses, which at least address some of the problems associated with the known products, compositions or agents identified above. It is an object of the present invention to provide such a product, composition or agent.

It has surprisingly been found that providing a horse with a supplement comprising a calcium coordination compound, such as chelated calcium, has a drastic effect on the overall temperament of many horses. It has been found that previously agitated horses have a calmer temperament. This effect improves the safety of the rider and the horse, and can increase the rider's enjoyment. Moreover, it has also been found that horses that train for and take part in equestrian sporting events are more alert, have a better concentration and a better reaction time. The latter effect has also been shown for calm horses.

The calcium coordination compound when provided in a sufficient amounts leads to a high proportion of the calcium being absorbed into a horse. Chelated calcium has sufficient bioavailability such that an ingestible amount can be provided to a horse and produces the calming effect.

A calcium coordination compound is calcium bonded to or associated with one or more ligands. Calcium coordination compounds include calcium chelates, which include calcium bonded to or associated with an organic molecule.
The calcium chelate can be bonded to or associated with amino acids, such as methionine, glycine, lysine etc.; carbohydrates such as gluconates, lactates, malates etc.; other organic molecules, such as citrate, ascorbate, acetate etc; and/or derived or concentrated from milk.

The calcium chelate may be selected from the following non-exhaustive list: calcium citrate, calcium gluconate, calcium citrate malate, calcium malate, calcium lactate, a calcium amino acid chelate, calcium aspartate, calcium ascorbate dihydrate, calcium aspartate, calcium boro gluconate, calcium bromolactobionate hexahydrate, calcium citratehydrate, calcium D-saccharate, calcium glubionate, calcium gluconate, calcium lactate gluconate, calcium lactobionate, calcium levulinate dihydrate, calcium magnesium lactate gluconate, calcium orotate dihydrate, calcium fumarate, calcium lactate, calcium succinate and/or calcium pidolate.

The calcium coordination compound may be bonded to or associated with protein, such as whey protein, casein, soy protein, pea protein, and cereal protein or other plant, algal, yeast, fungal, bacterial or animal derived proteins.

The calcium may be deliberately incorporated at high levels in living organisms such as plants, algae, yeasts, fungi, bacteria or single celled or multicellular animals

In a first aspect of the present invention, there is provided a supplement comprising or consisting essentially of a calcium coordination compound for use in a method of calming a horse according to claim 1. Preferably, the calcium coordination compound is one or more calcium chelates.

In a second aspect of the invention, there is provided a feed comprising the supplement according to claim 1. There is further provided a supplement for use in a method of calming a horse consisting of a calcium coordination compound only. Preferably, the calcium coordination compound is one or more calcium chelates.

Advantageously, the calcium chelate is selected from the group consisting of calcium citrate, calcium gluconate, calcium citrate malate, calcium malate, calcium lactate, a calcium amino acid chelate, calcium aspartate, calcium ascorbate dihydrate, calcium aspartate, calcium boro gluconate, calcium bromolactobionate hexahydrate, calcium citratehydrate, calcium D-saccharate, calcium glubionate, calcium gluconate, calcium lactate gluconate, calcium lactobionate, calcium levulinate dihydrate, calcium magnesium lactate gluconate, calcium orotate dihydrate, calcium fumarate, calcium succinate and/or calcium pidolate.

However, it is envisaged that any calcium chelate may be used. The supplement may comprise a single calcium chelate or two or more different calcium chelates, including two or more from the list above.

In a preferred embodiment of the invention, the calcium chelate is a calcium amino acid chelate, calcium gluconate and/or calcium citrate.

Advantageously, the calcium chelate is a calcium amino acid chelate and calcium gluconate; a calcium amino acid chelate and calcium citrate; or calcium gluconate and calcium citrate.

Further advantageously, the calcium chelate is a calcium amino acid chelate and calcium citrate malate; a calcium amino acid chelate and calcium malate; a calcium amino acid chelate and calcium lactate; a calcium amino acid chelate and calcium aspartate; calcium gluconate and calcium citrate malate; calcium gluconate and calcium malate; calcium gluconate and calcium lactate; calcium gluconate and calcium aspartate; calcium citrate and calcium citrate malate; calcium citrate and calcium malate; calcium citrate and calcium lactate; or calcium citrate and calcium aspartate. The above combinations may further comprise one or more additional calcium chelates from the list above.

As will be appreciated by those of skill in the art, the absorption rates of the individual calcium coordination compounds, including calcium chelates, will vary. The supplement is administered orally. It is intended that the supplement will be ingested by the horse. The supplement may be administered alone. The supplement may be administered with the feed; preferably the supplement is added to feed by the user or incorporated into feed or feed balancers when the feed is prepared/manufactured. Alternatively, the supplement may be supplied as a lick, as a drench from a container or a single dose drench administered by syringe. The load dose of the calcium (Ca++) in the supplement is greater than 1 gram of calcium per day, and preferably in the range of 1 - 20 grams per horse per day (based on a horse weighing 550 kgs). In an alternative embodiment the load dose is 10 to 15 grams of calcium per horse per day (based on a horse weighing 550 kgs), and preferably the load dose is 12 grams of calcium per horse per day (based on a horse weighing 550 kgs).

A skilled person will appreciate that the load dose is the amount of supplement ingested by a horse that is required to provide the horse with a calm temperament. In other words, the load dose is the amount of calcium present in the supplement. It is to be appreciated that the load dose may need to be administered from anything from one day to a number of months. The calming effect can be almost immediate. It has been found that commonly a horse's temperament will become calm over a two to three week period of receiving the load dose. However, the calming effect can take up to three months.

In the latter case, without being bound by any theory, it is considered that the horse in question has depleted stores of calcium (in the bones) and these stores remove calcium from the blood until they are replenished. It is not until these stores are replenished that consistent behaviour is achieved.

The actual amount of the supplement provided to the horse per day (the feed dose) will depend upon the calcium coordination complex/calcium chelate present in the supplement.

Advantageously, the feed dose for a 550kg horse is between 6 to 125 grams of supplement per day; 11 to 222 grams of supplement per day; or 5 to 95 grams of supplement per day.

In an embodiment in which the supplement comprises a calcium amino acid chelate, the feed dose for a 550 kilogram horse is 6 to 125 grams of the supplement per day. On this dosage 1 to 20 grams of calcium is available for absorption by the horse.

In an embodiment in which the supplement comprises calcium gluconate, the feed dose for a 550 kilogram horse is 11 to 222 grams of the supplement per day. On this dosage 1 to 20 grams of calcium is available for absorption by the horse.

In an embodiment in which the supplement comprises calcium citrate, the feed dose for a 550 kilogram horse is 5 to 95 grams of the supplement per day. On this dosage 1 to 20 grams of calcium is available for absorption by the horse.

Advantageously, the maintenance dose of the supplement is greater than 0.1 grams of calcium per day (based on a horse weighing 550kg). Preferably, the maintenance dose is the range of 0.1 - 8.0 grams of calcium per day, or more preferably in a range of 4 - 5 grams of calcium per day. A skilled person will appreciate that the maintenance dose is the amount of supplement ingested by the horse that is required to maintain the horse with a calm temperament after the initial state of agitation has been alleviated due to the ingestion of a load dose. In other words, the maintenance dose is the amount of calcium in the supplement that is administered to maintain the calmed temperament of the horse. The maintenance dose will depend upon the calcium coordination compound/calcium chelate present in the supplement. The maintenance dose will further depend upon the size and age of the horse.

Advantageously, the feed dose (including loading dose and/or maintenance dose) of the supplement is administered to a horse five days a week. These days may or may not be consecutive. The calcium supplement may be provided daily. However, the supplement has been found to be more effective when a single dosage five days a week is provided.

This administration is particularly recommended for mares at the time of foaling to avoid any potential risks such as increased probability of milk fever.

It is also envisaged that the calcium may be administered at a higher dose once or twice a week.

In advantageous embodiments of the invention, the supplement further comprises a marker. The purpose of the marker is to distinguish the supplement from other supplements in the market place as a security measure. The marker can be any compound capable of being identified in the supplement and safely administered to a horse; such compounds would be well known to those of skill in the art.

In one embodiment the marker is a dye. In one embodiment, the dye is canthaxanthin. In one embodiment the dye is a carotenoid. In one embodiment the marker is a fluorescent marker. In one embodiment the marker is a nutrient. In one embodiment the nutrient is a protein. In one embodiment the nutrient is a vitamin. In one embodiment the nutrient is a fat.

In one embodiment, the nutrient is a mineral. In one embodiment, the mineral is zinc. In one embodiment, the mineral is sulphur. In one embodiment, the mineral is boron. In one embodiment, the mineral is fluorine. In one embodiment, the mineral is silver. In one embodiment, the mineral is chronium. In one embodiment, the mineral is lithium. In one embodiment, the mineral is titanium. In one embodiment, the mineral is nickel. In one embodiment the marker is a glucosamine. In one embodiment, the marker is methylsulfonylmethane. In one embodiment the marker is a chondroitin. In one embodiment the marker is a vitamin C. In one embodiment the marker is a di methyl glycine. In one embodiment the marker is allicin. In one embodiment the marker is a L-carnitine.

The marker may be present in the amount of between 0.1 and 20% by weight of the supplement. The marker may be present in the amount of between 1 and 10% by weight of the supplement. The marker may be present in the amount of between 5 and 10% by weight of the supplement. The marker may be present in the amount of between 0.1 and 5% by weight of the supplement. It is to be appreciated that the supplement may include one or more of the above markers. The supplement is used in methods of calming horses; improving the behaviour, concentration and learning of a horse, improving judgement and decision making in competition horses; and/or alleviating psychological symptoms of calcium deficiency.

Although not wishing to be bound by any theory as to how the calcium coordination compound provides the calming effect, it is hypothesised that the agitated behaviour of the horses is due to a calcium deficiency that affects the transmission of nerve impulses and possibly causes excitability on the surface of the nerve cell impairing its function.

The invention will now be explained in more detail with respect to the following examples:
Three trials were carried out to investigate the effect of different calcium coordination compounds in the form of calcium chelates on horses demonstrating agitated behaviour and a fourth trial was carried out to demonstrate the effect of a non-coordinated calcium compound (non-chelated calcium) on horses demonstrating agitated behaviour. The four trials used calcium gluconate, a calcium amino acid chelate (MAAC Calcium 16 Amino Acid Chelate IPC from Albion Laboratories, Inc, USA), calcium citrate and calcium carbonate and the results are outlined below in examples 1 to 4 respectively.

The horses were given doses of calcium chelate calculated to provide a loading dose of 12 grams of calcium per day for five days each week for a period of a month. In the calcium carbonate subject the feed dose was 24 or 48 grams of calcium per day. The feeder provided a mark between 1 and 5 where bad is 1 and good is 5. The mark allocated was based on the effect noted in the behaviour of the horse. The feeder also provided general comments on the horses' behaviour during the trial.

### Example 1: Calcium gluconate

Three horses displaying agitated behaviour where provided with a supplement consisting of calcium gluconate only.

### Feeder 1

Feeder 1 provided a mark of 4.5. The horse was provided with 133 grams of calcium gluconate only. The feeder indicated that the horse calmed down without losing any energy and restored the horse to its behaviour prior to the agitated behaviour.

### Feeder 2

Feeder 2 provided a mark of 2.5. The horse was provided with 133 grams of calcium gluconate only. The feeder did not notice a difference in the horses' behaviour for the first couple of weeks. However, it was noted that the horse gained confidence. It is considered that the amount of the supplement needed to be increased for the specific horse.

### Feeder 3

Feeder three provided a mark of 3. The horse was provided with 133 grams of calcium gluconate only. The feeder indicated that an improvement in the horse's behaviour was noticed within three days and within two weeks the horse was completely calm.

The average mark for horses provided with calcium gluconate is 3.33.

### Example 2: Calcium amino acid chelate

Five horses displaying agitated behaviour where provided with a supplement consisting of a calcium amino acid chelate only.

### Feeder 1

Feeder 1 provided a mark of 5. The horse was provided with 75 grams of a calcium amino acid chelate only. The feeder indicated that the difference in the horse's behaviour was unbelievable. It was noted that the horse's concentration was much better. The horse went into the Wales qualifiers, a three day event, and coped very well with flood lights and loud noises.

### Feeder 2

Feeder 2 provided a mark of 5. The horse was provided with 75 grams of a calcium amino acid chelate only. The feeder indicated that the improvement in the horse's behaviour was brilliant. The horse calmed down within a few days.

### Feeder 3

Feeder 3 provided a mark of 2. The horse was with 75 grams of a calcium amino acid chelate only. The horse is a fussy eater. The feeder did not notice a change for the first couple of weeks, but in weeks three and four a change was noted although there were a couple of moments when the horse became agitated. It is considered that the horse was not receiving a sufficient amount of calcium because the horse is a fussy eater. It is postulated that an insufficient amount of calcium was ingested and hence absorbed by the horse.

### Feeder 4

Feeder 4 provided a mark of 3. The horse was provided with 75 grams of a calcium amino acid chelate only. The feeder noted that the horse was had a much calmer temperament in the first week. Unfortunately the trial was stopped after week one due to running out of the supplement.

### Feeder 5

Feeder 5 provided a mark of 3. The horse was provided with 75 grams of a calcium amino acid chelate only. The feeder noted that by the second week the horse was competing in (and won) an equine event. There was an improvement in the horse's ability to cope with noise and things going on around it.

The average mark for horses provided with calcium amino acid chelate is 3.60.

### Example 3: Calcium citrate

Three horses displaying agitated behaviour where provided with a supplement consisting of calcium citrate only.

### Feeder 1

Feeder 1 provided a mark of 4.5. The horse was provided with 57 grams of calcium citrate only. The feeder noted that the horse's behaviour was completely different. It was noted that the horse was a lot calmer and rational.

### Feeder 2

Feeder 2 provided a mark of 4. It was noted that the horse was much calmer and friendlier. The horse did have an episode of agitation but has subsequently been much calmer and no longer weaving or nodding head. The horse no longer gets nervous and protective of his space and is much calmer when new horses come into the yard.

### Feeder 3

Feeder 3 provided a mark of 5. The feeder noted that the horse's temperament showed a very good response. The horse was on box rest during the trial so it was not possible to test the horse's temperament and ability during ridden performance.

### Feeder 4

Feeder 4 provided a mark of 5. It was noted that the horse was much calmer generally. It was felt that the horse was much more willing to have a look at what is scaring her and was bolder with the handler. The horse was very calm, even when all the other horses around were being rather excitable during a hunt with lots of noise and commotion. It was also noticed that the horse's coat is incredibly shinny, which have never seen before.

The average mark for horses provided with calcium citrate is 4.6.

### Example 4: Calcium carbonate

Four horses displaying agitated behaviour where provided with a supplement consisting of calcium carbonate only.

### Feeder 1

Feeder 1 provided a mark of 1.5. The horse was provided with 60 grams of calcium carbonate only. It was noted that there was a not much difference in the behaviour of the horse.

### Feeder 2

Feeder 2 provided a mark of 2.5. The horse was provided with 120 grams of calcium carbonate only. The feeder noted a small improvement in the horse's behaviour but not sufficient for the horse to be ridden.

### Feeder 3

Feeder 3 provided a mark of 1. The horse was provided with 60 grams of calcium carbonate only. No significant change was noted in the horse's behaviour.

The average mark for horses provided with calcium carbonate is 1.67.

In comparison to the results of the chelated calcium trials, it is clear that non-chelated calcium is not readily absorbed by the horses and therefore does not produce a calming effect to horses.

The examples show that chelated calcium alone calms horses; improves behaviour, concentration and learning of a horse, improves judgement and decision making in competition horses; and appears to alleviate psychological symptoms of calcium deficiency.

Although the specific examples used supplements comprising calcium only, it is appreciated that the supplement can further comprise one or more components. It is appreciated that the horses will have a number of dietary requirements and, as such, a single supplement may be preferred.

The supplements described above may further comprise vitamin D. It is known that vitamin D is required for calcium absorption. It may be preferable to provide additional vitamin D, particularly when the horse does not have sufficient exposure to sunlight such as during winter months or when rugged or stabled for long periods of time.

The supplements described above may further comprise magnesium, sodium, potassium, tryptophan, B group vitamins, neurotransmitters and/or their precursors and/or herbal preparations. In one embodiment the supplement specifically does not contain a B group vitamin, such as thiamine (vitamin B1). In a further embodiment the supplement does not contain magnesium. In an alternative embodiment the supplement does not include magnesium or a B group vitamin, such as thiamine (vitamin B1).

In the present specification "comprises" means "includes or consists of" and "comprising" means "including or consisting of".

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

## Claims

1. A supplement comprising a calcium coordinated compound for use in a method of calming a horse, the method comprising administering the supplement to a horse, wherein the load dose is greater than 1 gram of calcium per day and the supplement is administered orally.

2. The supplement for the use according to claim 1, wherein the supplement consists essentially of a calcium coordinated compound.

3. The supplement for the use according to any one of claims 1 or 2, wherein the calcium coordinated compound is a calcium chelate; and preferably the calcium chelate is selected from the group consisting of calcium citrate, calcium gluconate, calcium citrate malate, calcium malate, calcium lactate, a calcium amino acid chelate, calcium aspartate, calcium ascorbate dihydrate, calcium aspartate, calcium boro gluconate, calcium bromolactobionate hexahydrate, calcium citratehydrate, calcium D-saccharate, calcium glubionate, calcium gluconate, calcium lactate gluconate, calcium lactobionate, calcium levulinate dihydrate, calcium magnesium lactate gluconate, calcium orotate dihydrate, calcium fumurate, calcium succinate and/or calcium pidolate.

4. The supplement for the use according claim 3, wherein the chelated calcium is a calcium amino acid chelate, calcium gluconate and/or calcium citrate.

5. The supplement for the use according to any one of the preceding claims, wherein the supplement is administered alone; or the supplement is administered with the feed; preferably the supplement is added to the feed by the user or incorporated into feed or feed balancers when the feed is prepared/manufactured.

6. The supplement for the use according to any one of the preceding claims, wherein the load dose is between 1 to 20 grams of calcium per day and/or the feed dose is between 6 to 125 grams of supplement per day; 11 to 222 grams of supplement per day; or 5 to 95 grams of supplement per day.

7. The supplement for the use according to any one of the preceding claims, wherein the loading dose is administered five days a week.

8. A feed comprising a supplement comprising a calcium coordinated compound for use in a method of calming a horse, the method comprising administering the feed to a horse, wherein the load dose is greater than 1 gram of calcium per day and the feed is administered orally.

## Patentansprüche

1. Nahrungsergänzungsmittel umfassend eine Calcium-koordinierte Verbindung zur Verwendung in einem Verfahren zum Beruhigen eines Pferdes, wobei das Verfahren das Verabreichen des Nahrungsergänzungsmittels an ein Pferd umfasst, worin die Belastungsdosis größer als 1 Gramm Calcium pro Tag ist und das Nahrungsergänzungsmittel oral verabreicht wird.

2. Nahrungsergänzungsmittel zur Verwendung nach Anspruch 1, worin das Nahrungsergänzungsmittel im Wesentlichen aus einer Calcium-koordinierten Verbindung besteht.

3. Nahrungsergänzungsmittel zur Verwendung nach einem der Ansprüche 1 oder 2, worin die Calcium-koordinierte Verbindung ein Calciumchelat ist; und das Calciumchelat vorzugsweise ausgewählt ist aus der Gruppe bestehend aus Calciumcitrat, Calciumgluconat, Calciumcitratmalat, Calciummalat, Calciumlactat, einem Calciumaminosäurechelat, Calciumaspartat, Calciumascorbatdihydrat, Calciumaspartat, Calciumborogluconat, Calciumbromolactobionathexahydrat, Calciumcitrathydrat, Calcium-D-saccharat, Calciumglubionat, Calciumgluconat, Calciumlactatgluconat, Calciumlactobionat, Calciumlevulinatdihydrat, Calciummagnesiumlactatgluconat, Calciumorotatdihydrat, Calciumfumarat, Calciumsuccinat und/oder Calciumpidolat.

4. Nahrungsergänzungsmittel zur Verwendung nach Anspruch 3, worin das chelierte Calcium ein Calciumaminosäurechelat, Calciumgluconat und/oder Calciumcitrat ist.

5. Nahrungsergänzungsmittel zur Verwendung nach einem der vorhergehenden Ansprüche, worin das Nahrungsergänzungsmittel allein verabreicht wird; oder das Nahrungsergänzungsmittel mit dem Futter verabreicht wird; das Nahrungsergänzungsmittel vorzugsweise vom Anwender dem Futter zugesetzt oder bei Zubereitung/Herstellung des Futters in Futter oder Feed Balancer einbezogen wird.

6. Nahrungsergänzungsmittel zur Verwendung nach einem der vorhergehenden Ansprüche, worin die Belastungsdosis zwischen 1 bis 20 Gramm Calcium pro Tag beträgt und/oder die Futterdosis zwischen 6 bis 125 Gramm Nahrungsergänzungsmittel pro Tag; 11 bis 222 Gramm Nahrungsergänzungsmittel pro Tag; oder 5 bis 95 Gramm Nahrungsergänzungsmittel pro Tag beträgt.

7. Nahrungsergänzungsmittel zur Verwendung nach einem der vorhergehenden Ansprüche, worin die Belastungsdosis fünf Tage pro Woche verabreicht wird.

8. Futter umfassend ein Nahrungsergänzungsmittel umfassend eine Calcium-koordinierte Verbindung zur Verwendung in einem Verfahren zum Beruhigen eines Pferdes, wobei das Verfahren das Verabreichen des Futters an ein Pferd umfasst, worin die Belastungsdosis größer als 1 Gramm Calcium pro Tag ist und das Futter oral verabreicht wird.

## Revendications

1. Un complément alimentaire contenant un composé coordonné du calcium destiné à être utilisé dans un procédé pour calmer un cheval, le procédé comprenant l'administration du complément à un cheval, la dose de charge étant supérieure à 1 gramme de calcium par jour et le complément étant administré par voie orale.

2. Le complément pour une utilisation selon la revendication 1, le complément étant essentiellement d'un composé coordonné du calcium.

3. Le complément pour une utilisation selon l'une quelconque des revendications 1 ou 2, le composé coordonné du calcium étant un chélate de calcium ; et le chélate de calcium étant sélectionné, de préférence, dans le groupe constitué par du citrate de calcium, du gluconate de calcium, du malate de citrate de calcium, du malate de calcium, du lactate de calcium, un chélate d'acide aminé de calcium, de l'aspartate de calcium, du dihydrate d'ascorbate de calcium, de l'aspartate de calcium, du borogluconate de calcium, de l'hexahydrate de bromolactobionate de calcium, de l'hydrate de citrate de calcium, du D-saccharate de calcium, du glubionate de calcium, du gluconate de calcium, du gluconate de lactate de calcium, du lactobionate de calcium, du dihydrate de lévulinate de calcium, du gluconate de lactate de calcium et magnésium, du dihydrate d'orotate de calcium, du fumarate de calcium, du succinate de calcium et/ou du pidolate de calcium.

4. Le complément pour une utilisation selon la revendication 3, le calcium chélaté étant un chélate d'acide aminé de calcium, du gluconate de calcium et/ou du citrate de calcium.

5. Le complément pour une utilisation selon l'une quelconque des revendications précédentes, le complément étant administré seul ; ou le complément étant administré avec l'aliment ; le complément étant, de préférence, ajouté à l'aliment par l'utilisateur ou incorporé à l'aliment ou aux aliments d'équilibrage lors de la préparation/fabrication de l'aliment.

6. Le complément pour une utilisation selon l'une quelconque des revendications précédentes, la dose de charge étant de 1 à 20 grammes de calcium par jour et/ou la dose alimentaire étant de 6 à 125 grammes de complément par jour ; de 11 à 222 grammes de complément par jour ; ou de 5 à 95 grammes de complément par jour.

7. Le complément pour une utilisation selon l'une quelconque des revendications précédentes, la dose de charge étant administrée cinq jours par semaine.

8. Un aliment contenant un complément comprenant un composé coordonné du calcium destiné à être utilisé dans un procédé pour calmer un cheval, le procédé comprenant l'administration de l'aliment à un cheval, la dose de charge étant supérieure à 1 gramme de calcium par jour et l'aliment étant administré par voie orale.
